# EUROPÄISCHE PATENTANMELDUNG

(11) **EP 0 745 580 A1**
(43) Veröffentlichungstag der Anmeldung: **04.12.1996**
(21) Anmeldenummer: 96107967.0
(22) Anmeldetag: 20.05.1996
(51) Int. Cl.: C07C 68/08, C07C 69/96

(54) **Verfahren zur Reinigung von Kohlensäurediarylestern unter Verwendung von Alumosilikaten**

(30) Priorität: 01.06.1995 DE 19520091
(71) Anmelder: BAYER AG, 51368 Leverkusen (DE)
(72) Erfinder: Kühling, Steffen, Dr., 40670 Meerbusch (DE); Bunzel, Lothar, 47906 Kempen (DE); Jeromin, Günther, Dr., 47802 Krefeld (DE); Zaby, Gottfried, Dr., 51375 Leverkusen (DE)

(57) **Zusammenfassung**

Die Erfindung betrifft ein Verfahren zur Reinigung von Kohlensäurediarylestern.

## Beschreibung

Die Erfindung betrifft ein Reinigungsverfahren von Kohlensäurediarylestern durch Behandlung mit Alumosilikaten und anschließende oder gleichzeitige Destillation.

Beim Einsatz von Kohlensäurediarylestern in das Polycarbonatumesterungsverfahren ist für eine gute Qualität des resultierenden Polycarbonats und für eine problemlose Reaktionsführung bzw. Katalyse des Prozesses eine gleichbleibend hohe Kohlensäurediarylesterqualität von Bedeutung. Daher wurde in der Vergangenheit die Reinigung der Kohlensäurediarylester durch Destillation (Encyclopedia of Polymer Science, Vol. 10 (1969), Chemistry and Physics of Polycarbonates, Polymer Reviews, H. Schnell, Vol. 9, John Wiley and Sons, Inc. (1964), S. 50/51) oder durch Behandlung mit einem Alkali-/Erdalkalimetallsalz und anschließender Destillation (GB-PS 1 096 936) durchgeführt.

Es wurde nun gefunden, daß durch Behandeln der Kohlensäurediarylester mit Alumosilikaten und anschließender Destillation bzw. während der Destillation eine Kohlensäurediarylesterqualität erzielt wird, die sich in besonderem Maße für den Einsatz in den Schmelzeumesterungsprozeß für die Polycarbonatherstellung eignet. Neben der guten Produktqualität zeichnet sich das Verfahren durch eine hohe Wirtschaftlichkeit aus, da der Reinigungsprozeß einfach und die Alumosilikate gut verfügbar sind.

Gegenstand der Erfindung ist ein Verfahren zur Reinigung von Kohlensäurediarylestern, dadurch gekennzeichnet, daß geschmolzenem Kohlensäurediarylester Alumosilikate zugesetzt werden und anschließend die Alumosilikate durch Filtration und/oder Destillation des Kohlensäurediarylesters durch eine gegebenenfalls mit Alumosilikaten beschickte Füllkörperkolonne abgetrennt werden.

Die erfindungsgemäß einzusetzenden Alumosilikate sind bekannt, z.B. Kirk-Othmer "Encyclopedia of Chemical Technology" 2nd Ed. 1964, Vol. 5 S. 541-561.

Eingesetzt werden können erfindungsgemäß z.B. Kaolin-Typen wie Kaolinit, Dickerit, Nacrit (alle Al₂O₃ x 2 SiO₂ x 2 H₂O) oder Anauxit (Al₂O₃ x 3 SiO₂ x 2 H₂O) oder Halloysit (Al₂O₃ x 2 SiO₂ x 2 H₂O) oder Endellit (Al₂O₃ x 2 SiO₂ x 4 H₂O) sowie die durch Erhitzen aus den Kaolintypen hergestellten Spinel-Typen, Serpentin-Typen, in denen 3 Mg-Ionen, 2 Al-Ionen - ausgehend von den Kaolin-Typen - ersetzt haben (Mg₃Si₂O₅(OH)₄). Zu den Serpentin-Typen gehören ferner Amesit (⁻(Mg₂Al)(SiAl)O₅(OH)₄) und Cronstedit (Fe₂²⁺Fe³⁺) (SiFe³⁺)O₅(OH₄) sowie Chamosit (Fe²⁺,Mg)_{2,3}(Fe³⁺Al)_{0,7}(Si_{1,14}Al_{0,86})O₅(OH)₄ sowie die zur Zeit auch synthetisch zugänglichen Nickel- oder Kobaltspezies.

Weiterhin können Alumosilikate des Montmorillonit-Typs eingesetzt werden wie
- Montmorillonit: [Al_{1,67}Mg_{0,33}(Na_{0,33})]Si₄O₁₀(OH)₂
- Beidellit: Al_{2,17}[Al_{0,33}(Na_{0,33})]Si_{3,17}O₁₀(OH)₂
- Nontronit: Fe³⁺[Al_{0,33}(Na_{0,33})]Si_{3,67}O₁₀(OH)₂
- Hectorit: Mg_{2,67}Li_{0,33}(Na_{0,33})]Si₄O₁₀(OH,F)₂
- Saponit: Mg_{3,0}[Al_{0,33}(Na_{0,33})]Si_{3,67}O₁₀(OH)₂
- Sauconit: [Zn_{1,48}Mg_{0,14}Al_{0,74}Fe³⁺][Al_{0,99}Si_{3,01}]O₁₀(OH)₂X_{0,33}
sowie Cu²⁺, Co²⁺ oder Ni²⁺ haltige Typen (X = Halogen) wie Volkoskoit, Medmontit oder Pimelit.

Derartige Tonerden können für sich oder als Gemisch von mehreren Tonerden verwendet werden. Sie können die für diese Naturprodukte üblichen Verunreinigungen enthalten (in Bentonit Montmorillonit-Reste, Feldspat, Quarz etc.).

Bevorzugt sind die als "Montmorillonit-Typen" beschriebenen Tonerden und Zeolithe, besonders bevorzugt Montmorillonit.

Bei den erfindungsgemäß einzusetzenden Zeolithen handelt es sich um kristalline, wasserhaltige Alumosilikate, synthetisiert oder natürlich vorkommend, mit Gerüststruktur (S. D.W. Breck in "Zeolite Molekular Sieves", Wiley Interscience, 1974, S. 133 bis 180; Ullmanns Enzyklopädie der technischen Chemie, 4. Aufl. Band 17, S. 9 bis 18, Verlag Chemie, Weinheim, New York). Bevorzugt sind solche der nachfolgenden Formel:

M_{2/n}O Al₂O₃ x SiO₂ YH₂O

in welcher
- M: für Protonen oder Metallkationen der Gruppe Ia, IIa, IIIa, IVa, Va, VIa, VIIa, VIIIa, Ib, IIb, IIIb und IVb, bevorzugt für Protonen der Metallkationen der Gruppe Ia, IIa, IVa und IVb des Periodensystems nach Mendelejew, besonders bevorzugt für Protonen und Ionen Na⁺, K⁺, Cs⁺, Ca⁺, Mg²⁺, Zn²⁺, La³⁺, Pr³⁺ und Ce³⁺ steht,
- n: für die Wertigkeit des Metallkations steht,
- x: für das Mol-Verhältnis SiO₂/Al₂O₃ steht, wobei x eine Zahl von 1,0 bis 50,0, bevorzugt 2,0 bis 25,0 sein kann und
- y: für eine Zahl von 0,1 bis 9 steht.

Geeignet für das erfindungsgemäße Verfahren sind Zeolithe der Struktur A und der Faujasitstruktur (Typ XUY), L. ZSM, Mordenit, Offretit, Phillipsit, Sodalith, ferner zeolithähnliche Materialien wie AlPO's, SAPO's und besonders geeignet sind Zeolithe der Struktur A der Faujasit-Struktur (X,Y-Typ), L, Mordenit, Offretit und (ferner SAPO's, AlPO's und MeApo's), ganz besonders geeignet sind Zeolithe der Struktur A der Faujasit-Struktur (X und Y) und Mordenit.

Kohlensäurediester im Sinne vorliegender Erfindung sind Di-C₆-C₂₀-Arylester, vorzugsweise die Diester von Phenol oder alkylsubstituierten Phenolen, z.B. Diphenylcarbonat, Dikresylcarbonat, bevorzugt Diphenylcarbonat (DPC).

Im erfindungsgemäßen Verfahren können Kohlensäurediester eingesetzt werden, die auf unterschiedlichste Art und Weise bzw. Verfahren hergestellt worden sind, z.B. solche die nach Phasengrenzflächenverfahren (Encyclopedia of Polymer Science, Vol. 10 (1969), Chemistry and Physics of Polycarbonates, Polymer Reviews, H. Schnell, Vol. 9, John Wiley and Sons, Inc. (1964)), lösungsmittelfreien Direktverfahren aus Monophenol und Carbonyldihalogenid (z.B. EP-A 483 632) nach lösungsmittelfreien, carbonyldihalogenidfreien Direktverfahren aus Kohlenmonoxid und Monophenol (z.B. DE-A 27 38 437), mittels des lösungsmittelfreien Umesterungsverfahren von Dialkylestern und Monophenolen (z.B. JP-A-291 257, JP-A-93 660) hergestellt wurden.

Die Behandlung bzw. der Kontakt der Kohlensäurediester mit den Alumosilikaten kann in der gasförmigen oder flüssigen Phase des Kohlensäurediesters erfolgen. Die Verweilzeit des Kohlensäurediesters mit dem Alumosilikat liegt zwischen dem Sekundenbereich z.B. beim Gaskontakt und bis zu mehreren Stunden, bevorzugt beim Flüssigkontakt bei 10 s bis 5 h, besonders bevorzugt zwischen 20 s und 1 h. Es besteht die Möglichkeit einer kontinuierlichen (Überleiten der Schmelze bzw. des Gases) oder diskontinuierlichen Reinigung. Die Behandlung des Kohlensäurediesters erfolgt bei Temperaturen im Bereich von 80°C bis 300°C, bevorzugt bei 100°C bis 250°C und einem Druck von 0,1 bis 1 bar, bevorzugt 0,1 bis 8 mbar.

Nach der Behandlung des Kohlensäurediesters mit dem Alumosilikat kann eine Destillation angeschlossen werden. Es kann vorteilhaft sein, die Behandlung des Kohlensäurediesters mit einer Destillation des Kohlensäurediesters zu koppeln. Bei der Destillation kann das Alumosilikat dann sowohl im Sumpf als auch im Gasraum eingesetzt werden; entsprechend unterschiedlich sind dann auch die, auf den Kohlensäurediarylester bezogen, einzusetzenden Gew.-% Alumosilikate, die von 0,01 Gew.-% (flüssig) bis über 99 Gew.-% (z.B. im Gasraum) reichen können, bevorzugt in der Flüssig- bzw. Schmelze-Fahrweise bei 0,1 Gew.-% bis 10 Gew.-%.

Die so gereinigten Kohlensäurediester zeichnen sich durch eine hohe Reinheit (GC > 99,9 %) und gutes Umesterungsverhalten aus (niedrige Katalysatorkonzentrationen (1 x 10⁻⁴ Mol-% Katalysator, z.B. NaOH, reichen für die Umesterung). Aus ihnen kann z.B. ein PC in guter Qualität hergestellt werden. Nicht nur das Umesterungsverhalten beim Einsatz in das Schmelzeumesterungsverfahren zur Herstellung von z.B. Polycarbonat verbessert sich, sondern auch die Möglichkeit der Abtrennung von organischen Verunreinigungen, wie z.B. Salicylsäurephenylester, Xanthon, 2-Phenoxybenzoesäure, chlorierten Arylcarbonaten, was sich in einer hohen GC-Reinheit widerspiegelt (>99,9 %).

Die Herstellung von aromatischen Oligo-/Polycarbonaten nach dem Schmelzeumesterungsverfahren ist bekannt, z.B. Encyclopedia of Polymer Science, Vol. 10 (1969), Chemistry and Physics of Polycarbonates, Polymer Reviews, H. Schnell, Vol. 9, John Wiley and Sons, Inc. (1964) oder dem US-A 5 340 905.

### Beispiele

### Beispiel 1

250 g Diphenylcarbonat (DPC) mit einer GC-Reinheit von >99,9 % wird mit 2 Gew.-% (5 g) Baylith TE 144 aufgeschmolzen und bei 8 mbar und 160°C über eine 40 cm-Füllkörperspiegelkolonne destilliert.

Das so erhaltene DPC wird dem Schmelze-PC-Eignungstest unterzogen. Das Umesterungsverhalten und somit die Eignung des Kohlensäurediphenylesters für die Schmelzeumesterung wird durch das Starttemperatur-Verfahren bestimmt. Dabei wird das Reaktionsgemisch, 17,1 g (0,075 Mol) 2,2-Bis-(4-hydroxyphenyl)-propan (DPA von der Shell) und der jeweilig zu prüfende (17,0 g, 0,07945 Mol) Kohlensäurediphenylester mit 0,0001 Mol-% NaOH (bezogen auf DPA), als 1 %ige wäßrige Lösung, katalysiert und in einem 100 ml Kolben mit Brücke und Thermometer in ein auf 270°C vorgeheiztes Ölbad eingesetzt. Die Sumpftemperatur und die Zeit die das Eduktgemisch zur Umesterung und somit zum Destillationsbeginn (Phenolabspaltung) benötigt, werden zum Vergleich notiert. Für ein gutes Umesterungsverhalten ist es notwendig, daß das DPC bereits mit geringen Konzentrationen Katalysator umestert. Dies ist dann gegeben, wenn bei dem obengenannten Starttemperaturverfahren die Sumpftemperatur <260°C bleibt und das erste abgespaltene Phenol nach <20 min destilliert.

Das nach dem obengenannten Verfahren gereinigte DPC zeigt nach 15 min und einer Sumpftemperatur von 255°C die erste Destillation, so daß das DPC zur Herstellung von Polycarbonat (PC) gut geeignet ist.

### Vergleichsbeispiel 1

Das Roh-DPC aus Beispiel 1 wird direkt dem Schmelze-PC-Eignungstest unterzogen. Selbst nach 60 min und dem Erreichen der Sumpftemperatur von 270°C ist noch keine Phenolbildung feststellbar. Das DPC ist zum Einsatz in den PC-Prozeß ungeeignet.

### Vergleichsbeispiel 2

Das Roh-DPC aus Beispiel 1 wird wie in Beispiel 1 beschrieben destilliert, nur wird kein Baylith zugesetzt. Jetzt wird es dem Schmelze-PC-Eignungstest unterzogen. Nach 60 min und dem Erreichen der Sumpftemperatur von 270°C ist noch keine Phenolbildung feststellbar. Das DPC ist zur Herstellung von Polycarbonat ungeeignet.

### Beispiel 2

Das Roh-DPC aus Beispiel 1 wird wie in Beispiel 1 beschrieben destilliert, nur ist die Spiegelkolonne mit 100 g Baylith TE 144 gefüllt, so daß das Diphenylcarbonat beim Destillieren über das Baylith strömt. Das so gereinigte DPC zeigt beim Schmelze-PC-Eignungstest nach 16 min und einer Sumpftemperatur von 257°C Phenolbildung. Das DPC ist zur Herstellung von Polycarbonat gut geeignet.

### Beispiel 3

Das Roh-DPC aus Beispiel 1 wird bei 180 bis 196°C über eine 40 cm-Säule (3 cm Durchmesser) gefüllt mit Baylith TE 144 geleitet und anschließend wie im Beispiel 1 beschrieben destilliert. Das so gereinigte DPC zeigt beim Schmelze-PC-Eignungstest nach 14 min und einer Sumpftemperatur von 258°C Phenolbildung. Das DPC ist zur Herstellung von Polycarbonat gut geeignet.

### Beispiel 4

250 g Roh-DPC (aus dem Direktphosgenierverfahren; Phenol + Phosgen an Aluminiumoxid) mit einer GC-Reinheit von 99,7 %, verunreinigt mit 0,25 % Salicylsäurephenylester, wird mit 2 Gew.-% (5 g) Baylith TE 144 aufgeschmolzen und das Diphenylcarbonat bei 8 mbar und 160°C über eine 40 cm-Füllkörperkolonne destilliert. Das so gereinigte DPC zeigt beim Schmelze-PC-Eignungstest nach 14 min und einer Sumpftemperatur von 258°C Phenolbildung. Darüber hinaus ist der Gehalt an Salicylsäurephenylester kleiner der Nachweisgrenze von 10 ppm. Das DPC ist zur Herstellung von Polycarbonat gut geeignet.

### Vergleichsbeispiel 3

Das Roh-DPC aus Beispiel 4 wird wie in Beispiel 4 beschrieben destilliert, nur wird kein Baylith zugesetzt. Jetzt wird es dem Schmelze-PC-Eignungstest unterzogen. Nach 60 min und dem Erreichen der Sumpftemperatur von 270°C ist noch keine Phenolbildung feststellbar. Darüber hinaus ist der Gehalt an Salicylsäurephenylester lediglich auf 0,12 % gesenkt. Das DPC ist zur Herstellung von Polycarbonat ungeeignet.

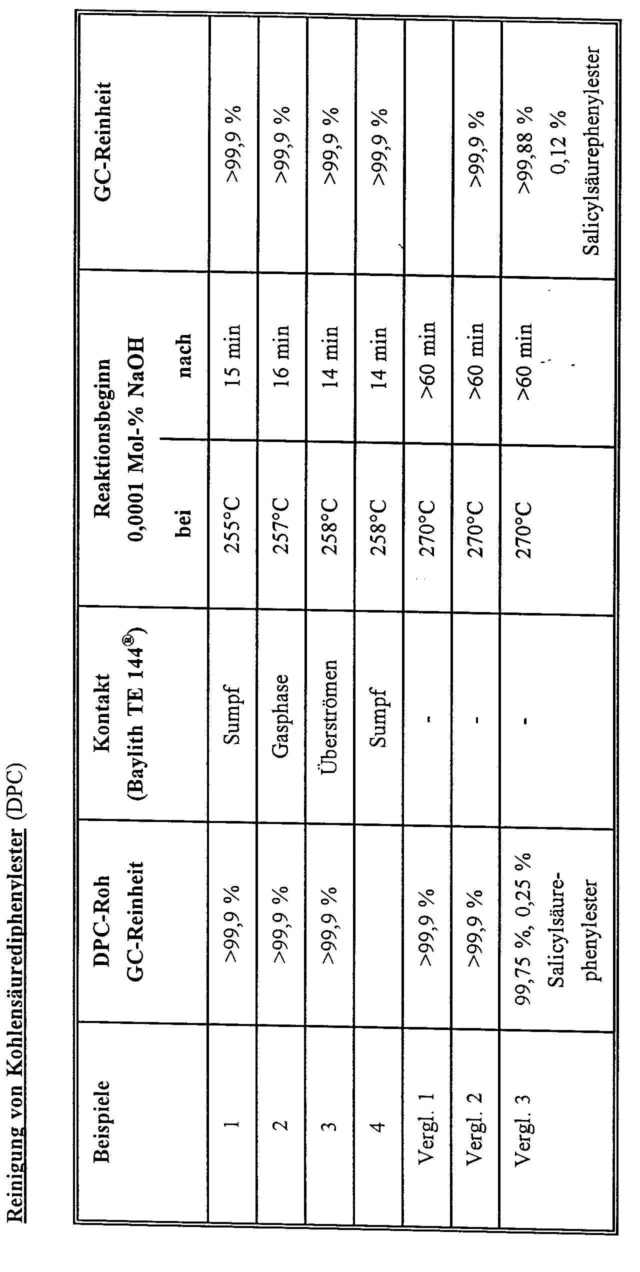

## Patentansprüche

1. Verfahren zur Reinigung von Kohlensäurediarylestern, dadurch gekennzeichnet, daß geschmolzenem Kohlensäurediarylester Alumosilikate zugesetzt werden und anschließend die Alumosilikate durch Filtration und/oder Destillation des Kohlensäurediarylesters durch eine gegebenenfalls mit Alumosilikaten beschickte Füllkörperkolonne abgetrennt werden.

2. Verwendung von nach Anspruch 1 gereinigtem Diphenylcarbonat zur Herstellung von Polycarbonat nach dem Schmelzumesterungsverfahren.
